# EUROPEAN PATENT APPLICATION

(11) **EP 4 397 319 A1**
(43) Date of publication of application: **10.07.2024**
(21) Application number: 22863203.0
(22) Date of filing: 22.08.2022
(51) Int. Cl.: A61K 47/68, C07D 491/22, C07K 1/18, C07K 1/22, A61P 35/00

(54) **METHOD FOR IMPROVING QUALITY OF ANTIBODY-DRUG CONJUGATE PRODUCT**

(30) Priority: 01.09.2021 CN 202111021482
(71) Applicant: Sichuan Kelun-Biotech Biopharmaceutical Co., Ltd., Chengdu, Sichuan 611138 (CN)
(72) Inventor: HU, Ruibin, CHENGDU, Sichuan 611138 (CN); SUN, Tao, CHENGDU, Sichuan 611138 (CN); WEN, Daihua, CHENGDU, Sichuan 611138 (CN); GE, Junyou, CHENGDU, Sichuan 611138 (CN); WU, Juan, CHENGDU, Sichuan 611138 (CN); WANG, Jing, CHENGDU, Sichuan 611138 (CN); WANG, Jingyi, CHENGDU, Sichuan 611138 (CN)
(74) Representative: Jones Day
(86) International application number: PCT/CN2022/113869
(87) International publication number: WO 2023/030064

(57) **Abstract**

The present application relates to a method for improving the product quality of an antibody-drug conjugate. The method comprises: conjugating a drug to be conjugated to an antibody to obtain an antibody-drug conjugate; and purifying the antibody-drug conjugate: a regulation solution is added to the antibody-drug conjugate, column chromatography flowthrough is performed to collect a product, and the product is further subjected to ultrafiltration and/or diafiltration concentration to obtain an antibody-drug conjugate with improved product quality. The obtained antibody-drug conjugate has high purity, a uniform DAR, no solvent residue, and stable quality. Moreover, the pretreatment of the antibody does not involve steps such as ultrafiltration and diafiltration, which simplifies antibody purification and reduces production costs.

## Description

The present application is based on the application with CN application No. 202111021482.5 filled on September 1, 2021, and claims its priority. The disclosure of the CN application is hereby incorporated herein in its entirety.

### TECHNICAL FIELD

The present application relates to the field of medicinal chemistry, and specifically to a method for improving the product quality of an antibody-drug conjugate.

### BACKGROUND

With the development of the field of antibody-drug conjugates, the development of production process conditions of purification and conjugation for biologically active drugs containing target antibodies is particularly important. The development and effective control of separation and purification processes for antibodies and a conjugation process for antibody-drug conjugates can ensure the product quality of drugs and are the key to the industrial production process of antibody-drug conjugates.

The design and parameter control of separation and purification process routes for antibodies directly affect the product quality and yield of antibody-drug conjugates. The existing purification process route of the antibody part of antibody-drug conjugates often requires including ultrafiltration and/or diafiltration steps to make the antibody meet the conjugation requirements. However, such steps increase the antibody purification steps and production costs, affecting the yields of the antibody and even the final conjugate.

On the other hand, the drug conjugation process has an important impact on the quality of antibody-drug conjugates. Currently, common conjugation methods for antibody-drug conjugates include: lysine conjugation, light and heavy chain reduced sulfide bond conjugation, and site-directed conjugation. Due to the diversity of conjugation sites and methods, for lysine conjugation, light and heavy chain reduced sulfide bond conjugation, or site-directed conjugation processes, the obtained antibody-drug conjugates are all accompanied by certain non-target products, and even occurrence of protein aggregation, affecting the pharmaceutical safety and effectiveness of the antibody-drug conjugates. The number of toxins attached to the antibody (drug-antibody ratio, DAR) determines the uniformity of the drug product. How to ensure product quality while controlling the DAR uniformity as much as possible is a difficulty in the production process of antibody-drug conjugate products. In the prior art, conjugation and purification processes of antibody-drug conjugates with DAR values between 3-4 are relatively common. Therefore, if purification and conjugation processes of antibody-drug conjugates with high DAR (for example, 6-8) can be developed, it is of great significance for improving product quality and reducing costs and safety risks.

### SUMMARY OF THE INVENTION

The inventors of the present application have conducted a large number of experiments and repeated explorations on the upstream antibody purification process and antibody-drug conjugation process of antibody-drug conjugates, and improved the reaction conditions and process parameters of each step in the production process. The method results in antibody products that can meet the conjugation requirements, improves the product purity and quality of final antibody-drug conjugates, significantly reduces the content of related substances and potential medication risks, and achieves stable and reliable product quality, thus being suitable for industrial continuous scale-up production.

The present invention provides a method for improving the product quality of an antibody-drug conjugate, comprising:
(1) providing an antibody-drug conjugate; and
(2) purifying the antibody-drug conjugate: a regulation solution is added to the antibody-drug conjugate, column chromatography flowthrough is performed to collect a product, and the product is further subjected to ultrafiltration and/or diafiltration concentration to obtain an antibody-drug conjugate with improved product quality;
   wherein the structure of the drug to be conjugated is shown in formula (I):

      D-[L₁-(L₂)ₘ₁-(L₃)ₘ₂-(L₄)ₘ₃-E]-G Formula (I)
   wherein,
   L₁ is 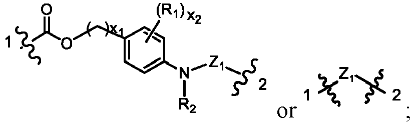 wherein, each R₁ and R₂ are independently hydrogen, halogen, carboxylate, sulfonate, cyano, C₁₋₆ alkyl, halogenated C₁₋₆ alkyl, cyano substituted C₁₋₆ alkyl (e.g., -CH₂CN), C₁₋₆ alkoxy, C₂₋₁₀ alkenyl, or C₂₋₁₀ alkynyl; Z₁ is an amino acid or a peptide consisting of 2 to 10 amino acids; x₁ and x₂ each are individually 0, 1, 2, 3, 4, 5, or 6; and L₁ is connected to D at position 1 and L₁ is connected to L₂ at position 2;
   L₂ is 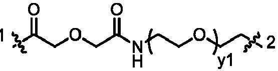 or absent; wherein, y₁ is 0, 1, 2, 3, 4, 5, 6, 7, 8, 9, or 10; and L₂ is connected to L₁ at position 1 and L₂ is connected to L₃ at position 2;
   L₃ is selected from a 5-12 membered heteroaromatic ring or absent;
   L₄ is 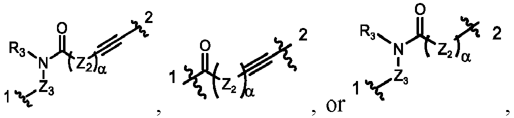 wherein Z₂ is selected from C₁₋₆ alkylene, C₂₋₁₀ alkenylene, C₂₋₁₀ alkynylene, C₃₋₈ cycloalkylene, and a 5-6 membered heteroaryl group; R₃ is selected from H and C₁₋₆ alkyl; Z₃ is absent or selected from C₁₋₆ alkylene; or, R₃ and Z₃, together with a nitrogen atom they are attached to, form a 4-8 membered heterocyclic group; α is 0, 1, 2, 3, 4, 5, or 6, and L₄ is connected to E at position 2 and L₄ is connected to L₃ at position 1;
   E is 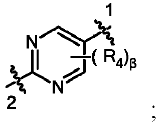 wherein, each R₄ is independently hydrogen, β is 0, 1 or 2, and E is connected to G at position 2 and E is connected to L₄ at position 1;
   m₁, m₂ and m₃ each are independently 0, 1, 2, 3, 4, 5, 6, 7, 8, 9, or 10;
   D is a biologically active molecule fragment; and
   G is a leaving group for nucleophilic substitution reaction; preferably, G is selected from halogen, sulfonyl, sulfonate, or nitro.

In certain embodiments, the present invention provides a method for improving the product quality of an antibody-drug conjugate, wherein a 5-20 mM reducing agent is added to an antibody that is pretreated and mixed uniformly for a reduction reaction, and then a drug to be conjugated is added, and after it is stirred and rested, an acidic solution is added;
a regulation solution is added to the product from the above step, column chromatography flowthrough is performed to collect a product, and the product is further subjected to ultrafiltration and/or diafiltration concentration to obtain an antibody-drug conjugate;
wherein the structure of the drug to be conjugated is shown in formula (I) mentioned above.

In certain embodiments, the antibody-drug conjugate in step (1) is obtained by the following method: a reducing agent solution with a concentration of 5-20 mM is added to an antibody that is pretreated, and then a drug to be conjugated is added to the reaction system, and after it is stirred and rested, an acidic solution is added to obtain the antibody-drug conjugate.

In certain embodiments, the method further includes the step of bacteria removal filtration.

In certain embodiments, the antibody is subjected to a bacteria removal filtration step prior to reduction reaction.

In certain embodiments, the reducing agent is tris(2-carboxyethyl)phosphine.

In certain embodiments, the reducing reaction includes treating the antibody with the reducing agent for 10 min-2h, for example, 30 min-1h.

In certain embodiments, the reduction reaction is treating the antibody with the reducing agent at room temperature; and/or, the reduction reaction is performed at 4-37°C, for example, 18-26°C. In certain embodiments, the column chromatography method is hydrophobic chromatography. Preferably, the hydrophobic chromatography includes steps of equilibration and elution.

In certain embodiments, the equilibrium step of the hydrophobic chromatography is using 20 mmol/L phosphate buffer-ammonium sulfate solution as an equilibrium solution under pH 6-7 conditions, wherein the conductivity is controlled at 50-80 mS/cm.

In certain embodiments, the equilibrium step of the hydrophobic chromatography is using 20 mmol/L phosphate buffer-ammonium sulfate solution as an equilibrium solution under pH 6-7 conditions, wherein the conductivity is controlled at 62-70 mS/cm.

In certain embodiments, the elution step of the hydrophobic chromatography is using the same solution as the equilibrium solution as an eluent under pH 6-7 conditions, wherein the conductivity is controlled at 22-30 mS/cm.

In certain embodiments, the chromatography column uses Butyl Sepharose High Performance packing.

In certain embodiments, the ultrafiltration and/or diafiltration concentration uses Pellicon 3 Cassette Biomax membrane.

In certain embodiments, a replacement solution used in the diafiltration is a 10 mmol/L histidine-histidine hydrochloride solution with a pH of 5.7-6.3.

In certain embodiments, the drug to be conjugated is added to the reaction system and stirred for 5-15 min, and then rested to react, preferably, rested for 1h-12h, such as 1h-6h, 1h-4h, 1h-3h, 1.5h-3h, or 1.5h-2.5h.

In certain embodiments, the stirring time is 5-15 min, the resting temperature is 4-37°C, for example, 18-26°C, and the resting time is 1.5-3 h.

In certain embodiments, the drug to be conjugated is a small molecular compound with a connecting arm. Preferably, the small molecular compound is selected from a DNA topoisomerase inhibitor, a tubulin inhibitor, or derivatives thereof.

In certain embodiments, the DNA topoisomerase inhibitor is a topoisomerase I inhibitor, and preferably, the topoisomerase I inhibitor is camptothecin, SN-38, irinotecan, topotecan, belotecan, rubitecan, or derivatives thereof.

In certain embodiments, the DNA topoisomerase inhibitor is a topoisomerase II inhibitor, and preferably, the topoisomerase II inhibitor is actinomycin D, doxorubicin, doxorubicin, docamicin, daunorubicin, mitoxantrone, podophyllotoxin, etoposide, or derivatives thereof.

In certain embodiments, the tubulin inhibitor is vinca alkaloid, vincristine, vinblastine, paclitaxel, docetaxel, cabazitaxel, or derivatives thereof.

In certain embodiments, the small molecular compound with a connecting arm is one selected from N-((S)-1-(((S)-1-((4-(((S)-2-((2R,3R)-3-((S)-1-((3R,4S,5S)-4-((S)-2-((S)-2-(dimethylamino)-3-methylbutylamido)-N,3-dimethylbutylamido)-3-methoxy-5-methylheptanoyl)pyrrolidin-2-yl)-3-methoxy-2-methylpropionylamino)-3-phenylpropionamido)methyl)phenyl)amino)-1-oxo-5-ureido-2-yl)amino)-3-methyl-1-oxobutan-2-yl)-6-(2-(methylsulfonyl)pyrimidin-5-yl)-5-hexyneamide;
(S)-4-ethyl-11-(2-(N-isopropylmethylsulfonamido)ethyl)-3,14-dioxo-3,4,12,14-tetrahydro-1H-pyrano[3',4',6,7]indolizino[1,2-b]quinolin-4-yl (4-((S)-42-(2-(methylsulfonyl)pyrimidin-5-yl)-4,8,37-trioxo-2-(3-ureidopropyl)-6,12,15,18,21,24,27,30,33-nonyloxy-3,9,36-azatetracosan-41-amido)benzyl)carbonate;
4-((S)-2-(4-aminobutyl)-42-(2-(methylsulfonyl)pyrimidin-5-yl)-4,8,37-trioxo-6,12,15,18,21,24,27,30,33-nonaoxa-3,9,36-triazadotetracontan-41-yneamido)benzyl-((S)-4-ethyl-11-(2-(N-isopropylmethanesulfonamido)ethyl)-3,14-dioxo-3,4,12,14-tetrahydro-1H-pyrano[3',4':6,7]indolizino[1,2-b]quinolin-4-yl)carbonate;
(S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl-4-((2S,5S)-5-isopropyl-2-methyl-38-(4-((6-(2-(methylsulfonyl)pyrimidin-5-yl)yl)hexan-5-yneamido)methyl)-1H-1,2,3-triazol-1-yl)-4,7,11-trioxo-9,15,18,21,24,27,30,33,36-nonyloxy-3,6,12-triazatriacontaneamido)benzylcarbonate;
4-((S)-2-(4-aminobutyl)-35-(4-((6-(2-(methylsulfonyl)pyrimidin-5-yl)hexan-5-yneamide)methyl)-1H-1,2,3-triazol-1-yl)-4,8-dioxo-6,12,15,18,21,24,27,30,33-nonaoxa-3,9-diazapentatriacontaneamido)benzyl((S)-4-ethyl-11-(2-(N-isopropylacetamido)ethyl)-3,14-dioxo-3,4,12,14-tetrahydro-1H-pyrano[3',4':6,7]indolizino[1,2-b]quinolin-4-yl)carbonate;
4-((S)-2-(4-aminobutyl)-35-(4-((6-(2-(methylsulfonyl)pyrimidin-5-yl)hexan-5-amido)methyl)-1H-1,2,3-triazol-1-yl)-4,8-dioxo-6,12,15,18,21,24,27,30,33-nonaoxa-3,9-diazapentatriacontaneamido)benzyl((S)-4-ethyl-11-(2-(N-isopropylmethylsulfonamido)ethyl)-3,14-dioxo-3,4,12,14-tetrahydro-1H-pyrano[3',4':6,7]indolizino[1,2-b] quinolin-4-yl)carbonate;
4-((S)-2-(4-aminobutyl)-35-(4-((2-(2-(methylsulfonyl)pyrimidin-5-yl)-oxazol-4-formamido)methyl)-1H-1,2,3-triazol-1-yl)-4,8-dioxo-6,12,15,18,21,24,27,30,33-nonyloxy-3,9-diazapentatriacontaneamido)benzyl-((S)-4-ethyl-11-(2-(N-isopropylmethylsulfonamido)ethyl)-3,14-dioxo-3,4,12,14-tetrahydro-1H-pyrano[3',4':6,7]indolizino[1,2-b] quinolin-4-yl)carbonate;
N-((1-((6S,9S)-1-amino-6-((4-(((S)-2-((2R,3R)-3-((S)-1-((3R,4S,5S)-4-((S)-2-((S)-2-(dimethylamino)-3-methylbutylamido)-N,3-dimethylbutylamido)-3-methoxy-5-methylheptanoyl)pyrrolidin-2-yl)-3-methoxy-2-methylpropionamide)-3-phenylpropionamide)methyl)phenyl)carbamoyl)-9-isopropyl-1,8,11,15-tetraoxo-13,19,22,25,28,31,34,37,40-nonaoxa-2,7,10,16-naphthotetrazin-42-yl)-1H-1,2,3-triazol-4-yl)methyl)-6-(2-(methylsulfonyl)pyrimidin-5-yl)-5-hexyneamide;
4-((2S,5S)-5-isopropyl-38-(4-((6-(2-(methylsulfonyl)pyrimidin-5-yl)-5-hexyneamide)methyl)-1H-1,2,3-triazol-1-yl)-4,7,11-trioxo-2-(3-ureidopropyl)-9,15,18,21,24,27,30,33,36-nonaoxa-3,6,12-triazadotetracontanyl)benzyl-((S)-1-(((S)-1-(((3R,4S,5S)-1-((S)-2-((1R,2R)-3-(((1S,2R)-1-hydroxy-l-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate;
(S)-2-((2R,3R)-3-((2S)-1-((3R,4S,5S)-4-((S)-N,3-dimethyl-2-((S)-3-methyl 2-(methyl(((4-((S)-2-((S)-3-methyl-2-(32-(4-((6-(2-(methylsulfonyl)pyrimidin-5-yl)hexan-5-carbamoyl)methyl)-1H-1,2,3-triazol-1-yl)-5-oxo-3,9,12,15,18,21,24,27,30-nonyloxy-6-azatriacontaneamido)butylamido)-5-ureidopentamido)benzyl)oxy)carbonyl)amino)butylamido)butylamido)-3-methoxy-5-methylheptyl)pyrrolidin-2-yl)-3-methoxy-2-methylpropionyl)-L-phenylalanine; and
4-((S)-2-(4-aminobutyl)-35-(4-((4-(2-(methylsulfonyl)pyrimidin-5-yl)benzamido)methyl)-1H-1,2,3-triazol-1-yl)-4,8-dioxo-6,12,15,18,21,24,27,30,33-nonyloxy-3,9-diazapentatriacontaneamido)benzyl ((S)-4-ethyl-11-(2-(N-isopropylmethylsulfonamido)ethyl)-3,14-dioxo-3,4,12,14-tetrahydro-1H-pyrano[3',4':6,7]indolizino[1,2-b] quinolin-4-yl)carbonate.

In certain embodiments, the drug to be conjugated is a small molecular compound with a connecting arm, and the small molecular compound with a connecting arm is selected from the compounds shown in the following structures: 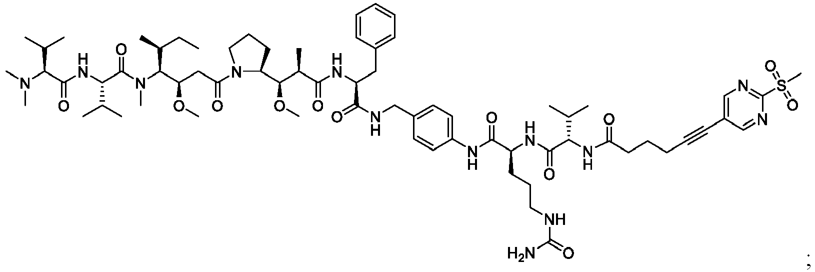 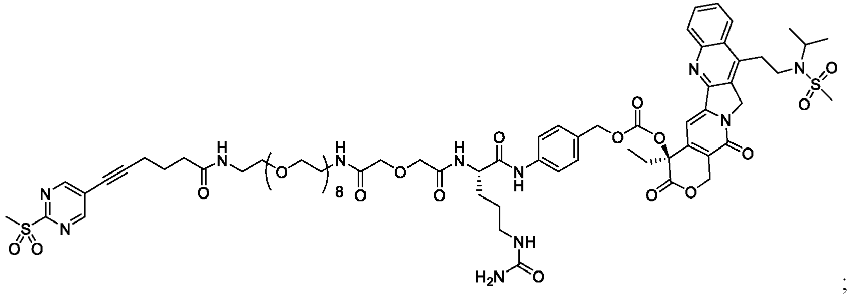 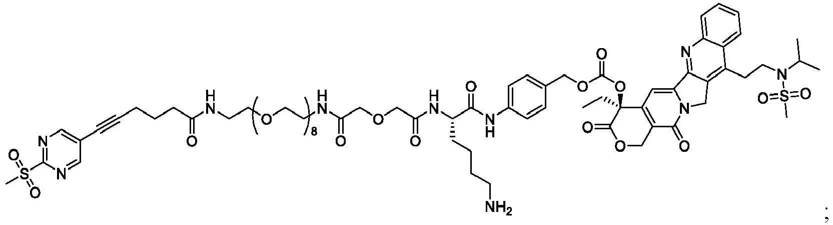 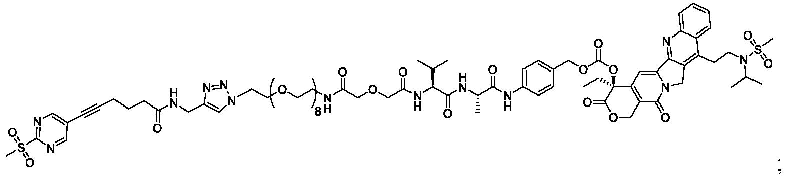 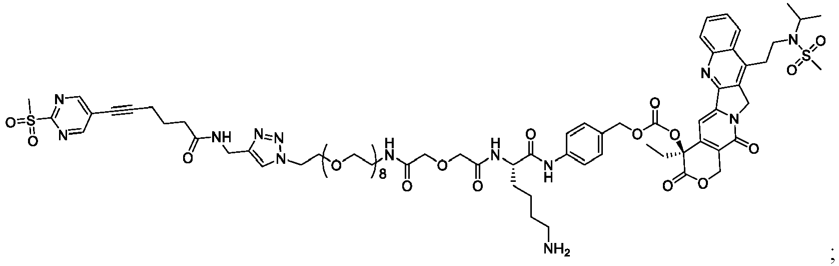 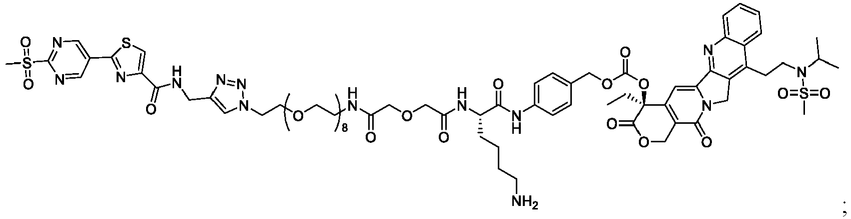 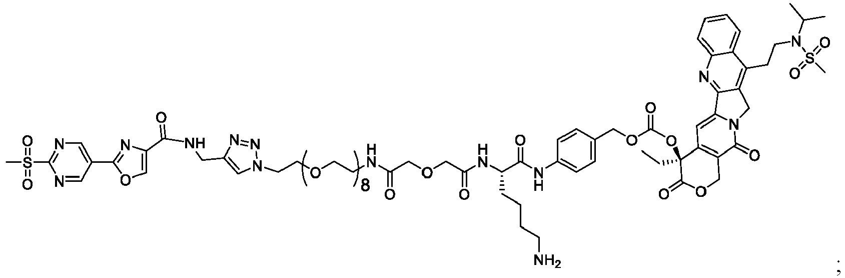 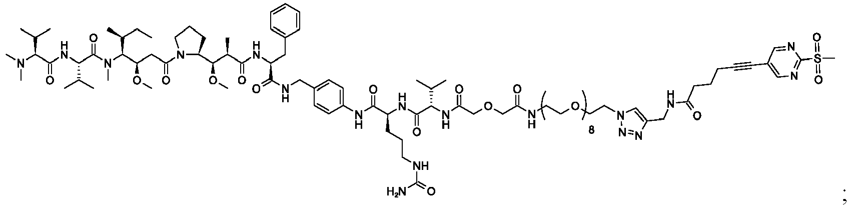 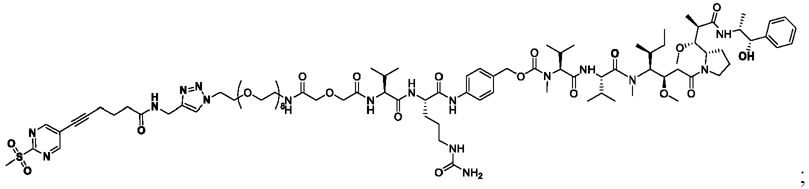 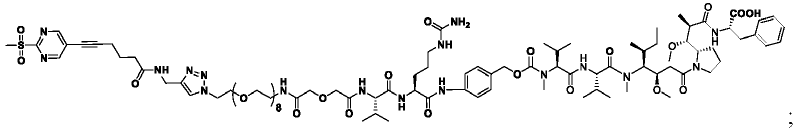 and

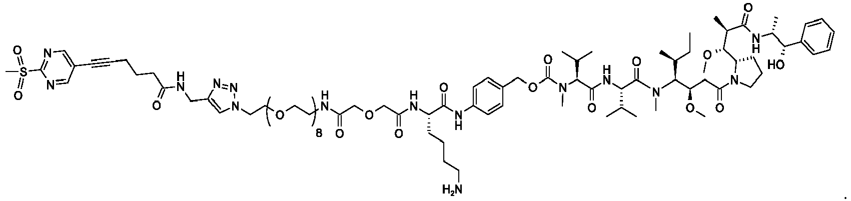

In certain embodiments, the antibody is Sacituzumab; the drug to be conjugated is a small molecular compound with a connecting arm, the structure of which is as follows:

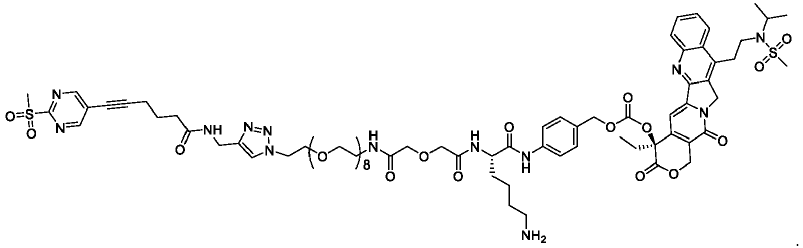

In some embodiments, the structure of the antibody-drug conjugate is as follows: 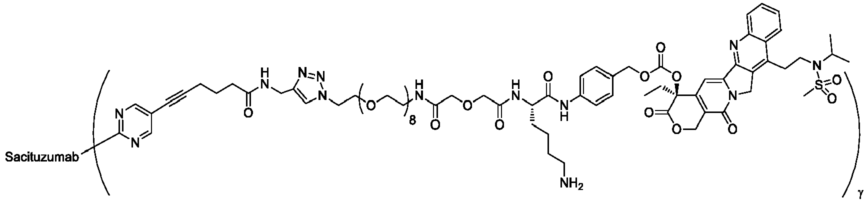 wherein, y is 1-10; preferably, y is 5-8; preferably, the connecting arm is connected to the sulfydryl group of Sacituzumab.

In certain embodiments, the dosing concentration of the drug to be conjugated is 10-70 mM, preferably 50 mM.

In certain embodiments, the pH value of the tris(2-carboxyethyl)phosphine solution is 7.0-8.0, for example, 7.4-8.0.

In certain embodiments, the acidic solution is selected from citric acid or acetic acid.

In certain embodiments, the acidic solution adjusts the pH of the reaction system to acidity; preferably, the acidic solution adjusts the pH of the reaction system to 6.0-7.0; further preferably, the acidic solution adjusts the pH of the reaction system to 4.5-7.0.

In certain embodiments, the antibody pretreatment includes steps of adding a disodium edetate solution to the antibody and adjusting the pH with a Tris solution. Preferably, the concentration of the disodium edetate solution is 2-10 mM, and the concentration of the Tris solution is 1-3 M; further preferably, the concentration of the disodium edetate solution is 2-10 mM, and the concentration of the Tris solution is 2 M.

In certain embodiments, the antibody pretreatment includes steps of adding a disodium edetate solution to the antibody and adjusting the pH using a phosphate. Preferably, the phosphate is sodium phosphate; further preferably, the sodium phosphate is disodium hydrogen phosphate; preferably, the concentration of the disodium edetate solution is 2-10 mM, and the concentration of the disodium hydrogen phosphate solution is 0.2-2 M, preferably 1 M.

In certain embodiments, the antibody is an anti-Her2 antibody, an anti-EGFR antibody, an anti-PD-1 antibody, an anti-PD-L1 antibody, or an anti-Trop-2 antibody.

In certain embodiments, the anti-Her2 antibody is Trastuzumab or Pertuzumab, the anti-EGFR antibody is Cetuximab or Nimotuzumab, the anti-PD-1 monoclonal antibody is Nivolumab or Pembrolizumab, the anti-PD-L1 monoclonal antibody is Atezolizumab or Durvalumab, and the anti-Trop-2 antibody is Sacituzumab or Datopotamab.

In certain embodiments, in the reduction reaction, the dosing concentration of the antibody is 10-25 g/L.

In certain embodiments, the regulation solution is selected from ammonium sulfate. In certain embodiments, the regulation solution is added to the antibody-drug conjugate prior to the hydrophobic chromatography step.

In certain embodiments, the regulation solution adjusts the antibody-drug conjugate to a high conductivity; preferably, the regulation solution adjusts the conductivity of the antibody-drug conjugate to 62-70 mS/cm.

In certain embodiments, the antibody is further purified prior to pretreatment.

In certain embodiments, the purification includes steps of chromatography selected from affinity chromatography, anion exchange chromatography, and/or cation exchange chromatography, and filtration.

In certain embodiments, the purification sequentially includes steps of affinity chromatography, incubation, depth filtration, anion exchange chromatography, and cation exchange chromatography.

In certain embodiments, the purification does not include ultrafiltration, diafiltration and the like.

In certain embodiments, a packing of the affinity chromatography is selected from Cytiva Mabselect Sure LX, Cytiva MabSelect SuRe, Cytiva MabSelect PrismA, Merck Eshmuno A, and Tosoh AF rProteinA-HC 650F.

In certain embodiments, the affinity chromatography further includes steps of equilibration, washing and elution. Preferably, the equilibration step uses NaCl-phosphate buffer; preferably, the washing can be performed at a pH of 5.4-6.2 for one, two or multiple times; preferably, the washing solution is selected from phosphate buffer-NaCl and sodium acetate-acetic acid solution; preferably, the elution uses sodium acetate-acetate buffer.

In certain embodiments, the elution step of the affinity chromatography requires controlling the pH at 3.5-3.7.

In certain embodiments, the incubation includes incubation at room temperature and pH 3.6-3.8.

In certain embodiments, the depth filtration uses Millipore XOHC.

In certain embodiments, a packing of the anion exchange chromatography is selected from Cytiva Q Sepharose Fast Flow, Thermo POROS 50HQ, Thermo POROS XQ, and Bio-Rad Nuvia HP-Q.

In certain embodiments, the pH of an operating system of the anion exchange chromatography is 5.5-6.5, preferably 5.8-6.2.

In certain embodiments, a packing of the cation exchange chromatography is selected from Tosoh Gigacap S 650M, Merck Eshmuno CPX, Thermo POROS 50HS, Thermo POROS XS, and Bio-Rad Nuvia HP-S.

In certain embodiments, the pH of an operating system of the cation exchange chromatography is 5.5-6.5, preferably 5.9-6.1.

In certain embodiments, the purification further includes steps of virus removal filtration and bacteria removal filtration.

The method of the present invention at least has the following beneficial effects:
(1) The present invention optimizes the details of process steps and reaction parameters, and uses hydrophobic chromatography and optimizes the parameters after the conjugation is completed, so that the antibody-drug conjugate obtained by the present invention has high purity, uniform DAR value, and almost undetectable residual solvent in the product, achieving controllability and stability of the product quality.
(2) The present invention performs purification and pretreatment on a conjugation antibody used in the antibody conjugation reaction. Conventional antibody pretreatment steps often require operations such as ultrafiltration and/or diafiltration. The present invention omits the above operations and controls process parameters of chromatography, filtration and the like, thereby reducing process time and cost and improving the overall yield of antibody products.

### DETAILED DESCRIPTION

The technical solutions in the embodiments of the present invention are clearly and fully described below with reference to the accompanying drawings in the embodiments of the present invention. It is clear that the described embodiments are merely some embodiments of the present invention rather than all the embodiments of the present invention. The following description of at least one exemplary embodiment is merely illustrative in nature and is in no way intended to limit the invention, and applications or uses thereof. Based on the embodiments of the present invention, all other embodiments obtained by those of ordinary skill in the art without making creative efforts fall within the scope of protection of the present invention. Unless otherwise specified, the reagents used in the following examples are commercially available products, and the solutions can be prepared using conventional techniques in the art.

### Example 1 Antibody pretreatment

### 1. Pretreatment of antibodies in batch 1

An affinity chromatography column (packing Mabselect Sure LX) was equilibrated with 20 mM phosphate buffer at pH 7.2 and 150 mM NaCl solution. The solution containing Sacituzumab antibody was loaded with a loading of 42.7 g/L. After completion, it was washed with at least 3 column volumes of 20 mM PB at pH 6.0 and 1M NaCl solution, and then washed a second time with at least 3 column volumes of 20 mM sodium acetate-acetic acid buffer at pH 5.5. After washing, it was eluted with 20 mM sodium acetate-acetic acid solution at pH 3.57.

The collected eluate was incubated at room temperature using citric acid to adjust to pH 3.7. After inactivation for 2 h, the resulting sample was neutralized with 2 M Tris. The above sample was clarified through depth filtration, with a loading of 1366 g/m² and Millipore X0HC as a membrane material. The treated sample was first subjected to anion exchange chromatography with a loading of 108.5 g/L, Cytiva Q Sepharose Fast Flow as packing, a loading conductivity of 2.68 mS/cm, and a pH of 6.09. The flow-through peak was collected after loading. The flow-through sample was chromatographed with a cationic column packed with Gigacap S 650M, a loading of 49.2 g/L, an elution pH of 6.02, and 20 mM PB+105 mM sodium chloride solution as eluent. The product was collected and used in the next step. The collected product was prefiltered and then subjected to virus removal filtration and bacteria removal filtration using Millipore Viresolve Pro to obtain the final pretreated antibody.

### 2. Pretreatment of antibodies in batch 2

An affinity chromatography column (packing Mabselect Sure LX) was equilibrated with 20 mM phosphate buffer at pH 7.2 and 150 mM NaCl solution. The solution containing Sacituzumab antibody was loaded with a loading of 41.9 g/L. After completion, it was washed with at least 3 column volumes of 20 mM PB at pH 6.0 and 1M NaCl solution, and then washed a second time with at least 3 column volumes of 20 mM sodium acetate-acetic acid buffer at pH 5.5. After washing, it was eluted with 20 mM sodium acetate-acetic acid solution at pH 3.62.

The collected eluate was incubated at room temperature using citric acid to adjust to pH 3.69. After inactivation for 2 h, the resulting sample was neutralized with 2 M Tris solution. The above sample was clarified through depth filtration, with a loading of 1394.9 g/m² and Millipore X0HC as a membrane material. The treated sample was first subjected to anion exchange chromatography with a loading of 107.7 g/L, GE Q Sepharose Fast Flow as packing, a loading conductivity of 2.71 mS/cm, and a pH of 6.07. The flow-through peak was collected after loading. The flow-through sample was chromatographed with a cationic column packed with Gigacap S 650M, a loading of 48.6 g/L, an elution pH of 6.03, and 20 mM PB+105 mM sodium chloride solution as eluent. The product was collected and used in the next step. The collected product was prefiltered and then subjected to virus removal filtration and bacteria removal filtration using Millipore Viresolve Pro to obtain the final pretreated antibody.

### 3. Pretreatment of antibodies in batch 3

An affinity chromatography column (packing Mabselect Sure LX) was equilibrated with 20 mM phosphate buffer at pH 7.2 and 150 mM NaCl solution. The solution containing Sacituzumab antibody was loaded with a loading of 43.2 g/L. After completion, it was washed with at least 3 column volumes of 20 mM PB at pH 6.0 and 1M NaCl solution, and then washed a second time with at least 3 column volumes of 20 mM sodium acetate-acetic acid buffer at pH 5.5. After washing, it was eluted with 20 mM sodium acetate-acetic acid solution at pH 3.58.

The collected eluate was incubated at room temperature using citric acid to adjust to pH 3.66. After inactivation for 2 h, the resulting sample was neutralized with 2 M Tris solution. The above sample was clarified through depth filtration, with a loading of 1447.8 g/m² and Millipore X0HC as a membrane material. The treated sample was first subjected to anion exchange chromatography with a loading of 116 g/L, GE Q Sepharose Fast Flow as packing, a loading conductivity of 2.69 mS/cm, and a pH of 6.06. The flow-through peak was collected after loading. The flow-through sample was chromatographed with a cationic column packed with Gigacap S 650M, a loading of 52.8 g/L, an elution pH of 6.0, and 20 mM PB+105 mM sodium chloride solution as eluent. The product was collected and used in the next step. The collected product was prefiltered and then subjected to virus removal filtration and bacteria removal filtration using Millipore Viresolve Pro to obtain the final pretreated antibody.

Samples collected in each step of the above three batches were tested for IEC purity using ion exchange chromatography and for SEC purity using SEC-HPLC method. The results are shown in the table below.

**Table 1 Purity test results**

| Sample | IEC (main peak%) | | | SEC (monomer%) | | |
|---|---|---|---|---|---|---|
| | Batch 1 | Batch 2 | Batch 3 | Batch 1 | Batch 2 | Batch 3 |
| Affinity elution sample | 77.2 | 76.3 | 76.6 | 98.9 | 98.4 | 98.6 |
| Depth filtration sample | 77.6 | 77.0 | 76.3 | 99.0 | 99.1 | 99.1 |
| Anion flow-through sample | 77.4 | 76.8 | 76.3 | 99.0 | 99.1 | 98.9 |
| Cation elution sample | 77.7 | 77.2 | 76.5 | 99.0 | 99.2 | 99.0 |
| Final product | 78.2 | 77.4 | 76.4 | 98.7 | 99.2 | 99.1 |

It can be seen from Table 1 that the three batches of antibodies produced by the process of the present invention all meet the requirements in terms of the purity indicators and can be used for the subsequent preparation of antibody-drug conjugates.

Samples collected in each step of the above three batches were tested for DNA residue and HCP residue. Residual DNA was tested using fluorescence quantitative PCR and residual HCP was tested using ELISA. The results are shown in the table below.

**Table 2 Residual substance test results**

| Sample | DNA residue (pg/mg) | | | HCP residue (ppm) | | |
|---|---|---|---|---|---|---|
| | Batch 1 | Batch 2 | Batch 3 | Batch 1 | Batch 2 | Batch 3 |
| Affinity elution sample | 16.3 | 13.0 | 15.9 | 1660.4 | 1705.1 | 1643.9 |
| Depth filtration sample | <0.19 | <0.18 | <0.18 | 12.8 | 24.0 | 12.1 |
| Anion flow-through sample | <0.35 | <0.34 | <0.33 | 5.6 | 4.9 | 4.1 |
| Cation elution sample | <0.16 | <0.16 | <0.16 | 3.6 | 2.7 | 5.3 |
| Final product | <0.19 | <0.19 | <0.18 | 2.8 | 1.6 | 3.7 |

It can be seen from Table 2 that the three batches of antibodies produced by the process of the present invention all meet the requirements in terms of DNA residue and HCP residue and can be used for the subsequent preparation of antibody-drug conjugates.

### Example 2 Preparation of antibody-drug conjugates

Sacituzumab antibody was diluted with 0.25 mL of a solution (pH 7.6) containing 20 mM PB, 150 mM NaCl and 5 mM disodium edetate to adjust the pH to 7.4, 10 mM TCEP was added as a reducing agent for conjugation of a drug to be conjugated and the Sacituzumab antibody. The two was mixed uniformly and rested at room temperature for 2 h. After completion, cysteine was added to terminate the reaction. Finally, the buffer was replaced using G-25 gel column to obtain the conjugated product.

In this example, the drugs to be conjugated as shown in the following structures were respectively conjugated with Sacituzumab to obtain antibody-drug conjugates:

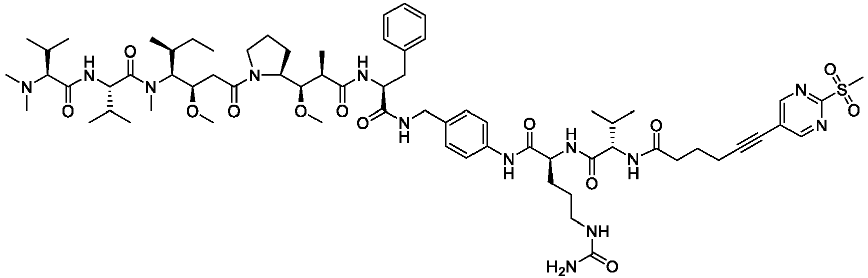

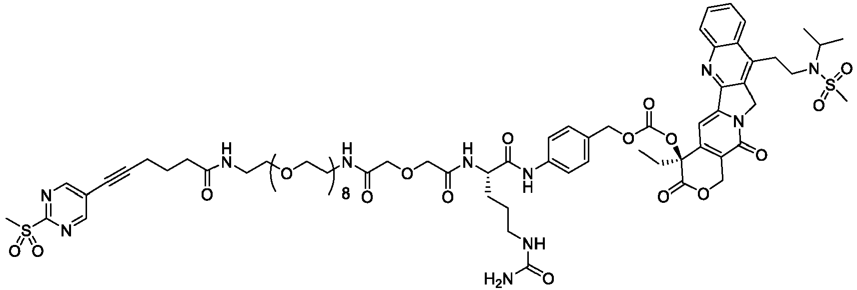

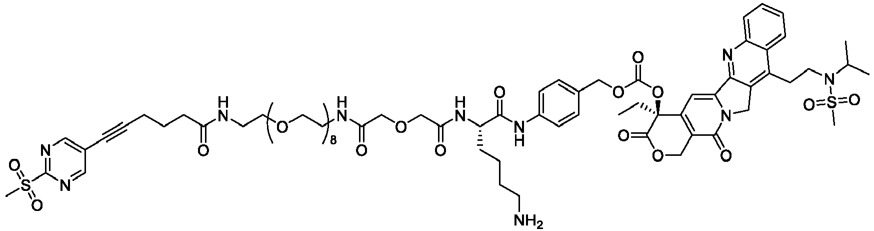

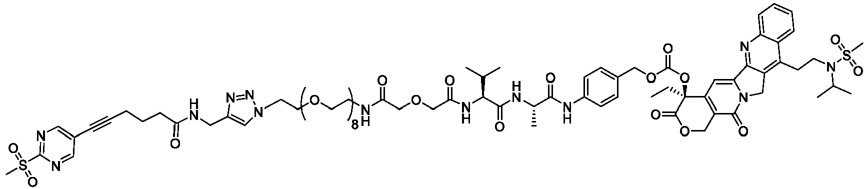

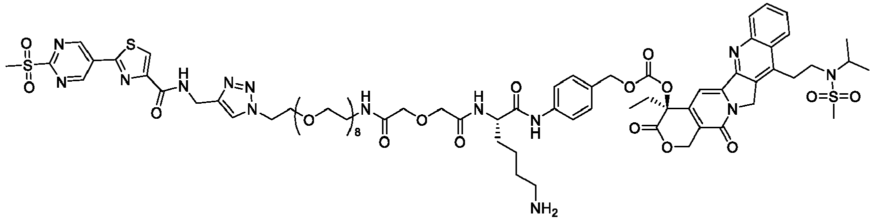

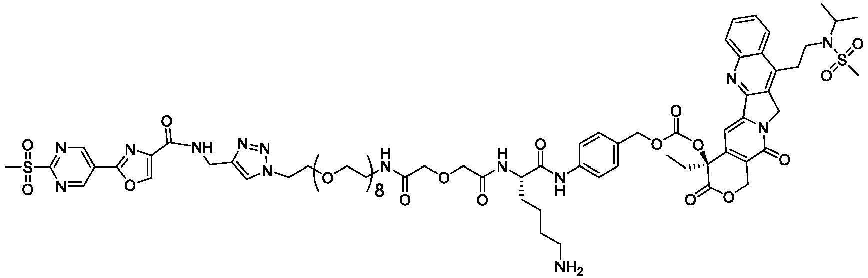

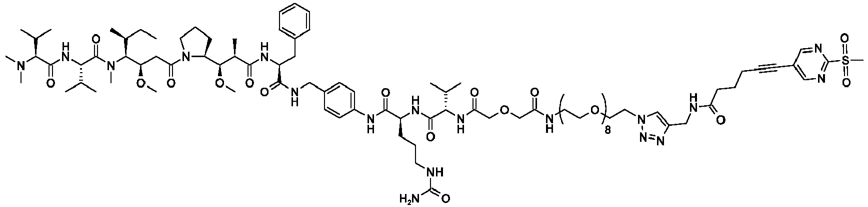

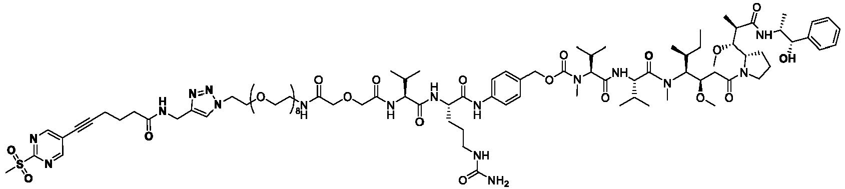

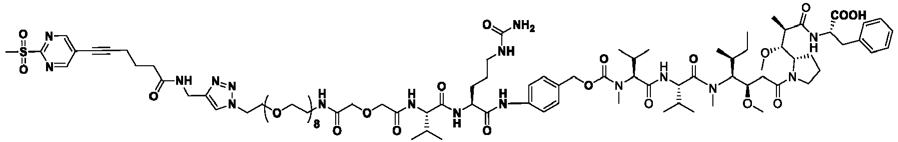

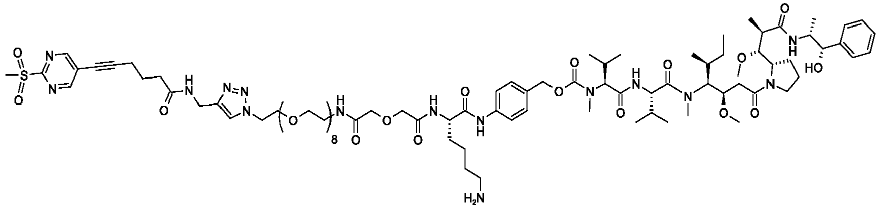

### Example 3 Improvement of the product quality of antibody-drug conjugates

A 5 mM disodium edetate regulation solution was added to a slowly stirred pretreated antibody to be conjugated (prepared according to the method of Example 1), and 2M Tris was used to adjust the pH concentration to alkaline. 10 mM TCEP solution was added to the antibody solution and mixed evenly with stirring. After reacting for 35 min at 23.3°C, a 50 mM solution of a drug to be conjugated was added to the reaction system, mixed evenly with stirring, and rested at room temperature. After reacting for 150 min, 1 M citric acid was used to adjust the pH to acidic (pH 6.0-7.0) to obtain the target product. Herein, the structural formula of the drug to be conjugated is

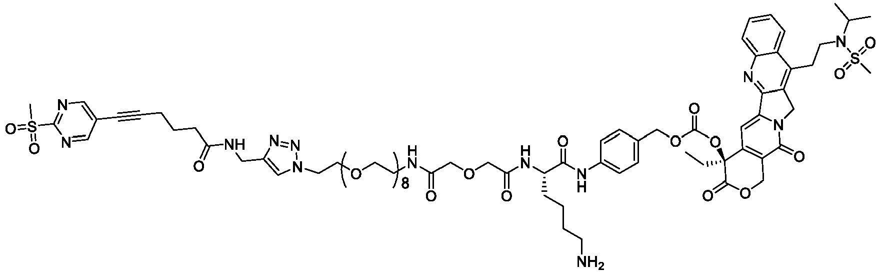

Ammonium sulfate regulation solution was added into the target product, and then hydrophobic column chromatography flowthrough was performed to collect a product, where the hydrophobic chromatography column packing was Butyl Sepharose High Performance, the equilibrium solution was a 20 mmol/L phosphate buffer-ammonium sulfate solution at pH 6.59, and conductivity was 66.51 mS/cm. The same eluent as the equilibrium solution was selected for elution, where conductivity was 26.93 mS/cm. The product of hydrophobic chromatography was further concentrated by ultrafiltration to obtain an antibody-drug conjugate.

The intermediates in each step of the above preparation were collected, and tested for purity using SEC-HPLC method, DAR using RP-HPLC method, free toxin using RP-HPLC method, and bacterial endotoxin according to the general rule 1143 of Chinese Pharmacopoeia, 2015 edition - Gel method. The results are shown in the table below.

**Table 3 Test results of purity, DAR, free toxin and bacterial endotoxin for conjugation intermediates**

| Intermediate name | SEC (monomer%) | DAR | Free toxin (%, mol/mol small molecular compound with a connecting arm/small molecular compound) | Endotoxin (EU/ml) |
|---|---|---|---|---|
| Conjugated sample | 98.6 | 7.4 | 1.4/6.7 | Not detected |
| Hydrophobic chromatography elution sample | 99.3 | 7.4 | 3.5/1.4 | Not detected |
| Sample after ultrafiltration replacement | 99.8 | 7.4 | 0.5/0.1 | <0.64 |

As can be seen from Table 3, the antibody-drug conjugate produced by the conjugation process of the present invention has high purity, uniform DAR value, and effective control of free toxin and endotoxin contents, which significantly improves the product quality of the prepared antibody-drug conjugate.

### Example 4 Improvement of the product quality of antibody-drug conjugates

A 5 mM disodium edetate regulation solution was added to a slowly stirred pretreated antibody to be conjugated (prepared according to the method of Example 1), and 2M Tris was used to adjust the pH concentration to alkaline. 10 mM TCEP solution was added to the antibody solution and mixed evenly with stirring. After reacting for 32 min at 23.5°C, a 50 mM solution of a drug to be conjugated was added to the reaction system, mixed evenly with stirring, and rested at room temperature. After reacting for 2.5 h, 1 M citric acid was used to adjust the pH to acidic (pH 6.0-7.0) to obtain the target product. Herein, the structural formula of the drug to be conjugated is

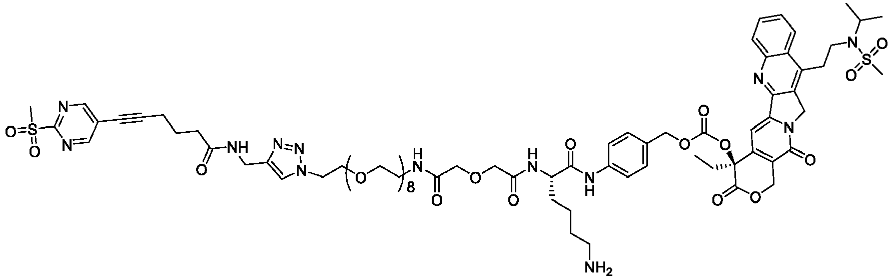

Ammonium sulfate regulation solution was added into the target product, and then hydrophobic column chromatography flowthrough was performed to collect a product, where the hydrophobic chromatography column packing was Butyl Sepharose High Performance (GE), the equilibrium solution was a 20 mmol/L phosphate buffer-ammonium sulfate solution at pH 6.59, and conductivity was 66.69 mS/cm. The eluent was the same as the equilibrium solution and conductivity was 27.16 mS/cm. The product of hydrophobic chromatography was further concentrated by ultrafiltration to obtain an antibody-drug conjugate.

The intermediates in each step of the above preparation were tested for purity, DAR, free toxin and bacterial endotoxin (the test method was the same as in Example 3), and the results are as shown in the table below.

**Table 4 Test results of purity, DAR, free toxin and bacterial endotoxin for conjugation intermediates**

| Intermediate name | SEC (monomer%) | DAR | Free toxin (%, mol/mol small molecular compound with a connecting arm/small molecular compound) | Endotoxin (EU/ml) |
|---|---|---|---|---|
| Conjugated sample | 98.5 | 7.3 | 1.7/9.3 | Not detected |
| Hydrophobic chromatography elution sample | 99.6 | 7.3 | 1.6/1.2 | Not detected |
| Sample after ultrafiltration replacement | 99.7 | 7.3 | Not detected | <0.64 |

As can be seen from Table 4, the antibody-drug conjugate produced by the conjugation process of the present invention has high purity, uniform DAR value, and effective control of free toxin and endotoxin contents, which significantly improves the product quality of the prepared antibody-drug conjugate.

### Example 5 Improvement of the product quality of antibody-drug conjugates

A 5 mM disodium edetate regulation solution was added to a slowly stirred pretreated antibody to be conjugated (prepared according to the method of Example 1), and 2 M Tris was used to adjust the pH concentration to alkaline. 10 mM TCEP solution was added to the antibody solution and mixed evenly with stirring. After reacting for 35 min at 22.3°C, a 50 mM solution of a drug to be conjugated was added to the reaction system, mixed evenly with stirring, and rested at room temperature. After reacting for 153 min, 1 M citric acid was used to adjust the pH to acidic (pH 6.0-7.0) to obtain the target product. Herein, the structural formula of the drug to be conjugated is

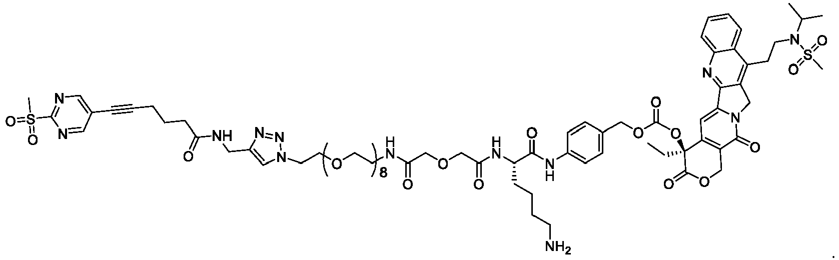

Ammonium sulfate regulation solution was added into the target product, and then hydrophobic column chromatography flowthrough was performed to collect a product, where the hydrophobic chromatography column packing was Butyl Sepharose High Performance (GE), the equilibrium solution was a 20 mmol/L phosphate buffer-ammonium sulfate solution at pH 6.51, and conductivity was 66.66 mS/cm. The eluent was the same as the equilibrium solution and conductivity was 26.98 mS/cm. The product of hydrophobic chromatography was further concentrated by ultrafiltration to obtain an antibody-drug conjugate.

The intermediates in each step of the above preparation were tested for purity, DAR, free toxin and bacterial endotoxin (the test method was the same as in Example 3), and the results are as shown in the table below.

**Table 5 Test results of purity, DAR, free toxin and bacterial endotoxin for conjugation intermediates**

| Intermediate name | SEC (monomer%) | DAR | Free toxin (%mol/%mol small molecular compound with a connecting arm/small molecular compound) | Endotoxin (EU/ml) |
|---|---|---|---|---|
| Conjugated sample | 98.0 | 7.3 | 1.5/6.0 | Not detected |
| Hydrophobic chromatography elution sample | 99.7 | 7.3 | 1.1/0.8 | Not detected |
| Sample after ultrafiltration replacement | 99.8 | 7.3 | Not detected | <0.64 |

As can be seen from Table 5, the antibody-drug conjugate produced by the conjugation process of the present invention has high purity, uniform DAR value, and effective control of free toxin and endotoxin contents, which significantly improves the product quality of the prepared antibody-drug conjugate.

The above descriptions are only preferred embodiments of the present invention and are not intended to limit the present invention. Any modifications, equivalent substitutions, improvements, etc. made within the spirit and principles of the present invention shall be included in the scope of protection of the present invention. In addition, the technical solutions between the various embodiments can be combined to each other, but this must be based on the ability of ordinary skilled in the art to achieve. When a combination of technical solutions conflicts or cannot be achieved, it should be considered that this combination of technical solutions does not exist, nor is it within the scope of protection claimed by the present invention.

## Claims

1. A method for improving the product quality of an antibody-drug conjugate, comprising:
(1) conjugating a drug to be conjugated to an antibody to obtain an antibody-drug conjugate; and
(2) purifying the antibody-drug conjugate: a regulation solution is added to the antibody-drug conjugate, column chromatography flowthrough is performed to collect a product, and the product is further subjected to ultrafiltration and/or diafiltration concentration to obtain an antibody-drug conjugate with improved product quality;
wherein the structure of the drug to be conjugated is shown in formula (I):
D-[L₁-(L₂)ₘ₁-(L₃)ₘ₂-(L₄)ₘ₃-E]-G Formula (I)
wherein,
L₁ is 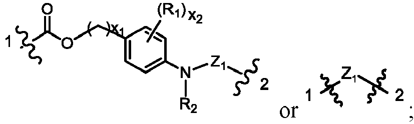 wherein, each R₁ and R₂ are independently hydrogen, halogen, carboxylate, sulfonate, cyano, C₁₋₆ alkyl, halogenated C₁₋₆ alkyl, cyano substituted C₁₋₆ alkyl (e.g., -CH₂CN), C₁₋₆ alkoxy, C₂₋₁₀ alkenyl, or C₂₋₁₀ alkynyl; Z₁ is an amino acid or a peptide consisting of 2 to 10 amino acids; x₁ and x₂ each are individually 0, 1, 2, 3, 4, 5, or 6; and L₁ is connected to D at position 1 and L₁ is connected to L₂ at position 2;
L₂ is 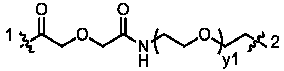 or absent; wherein, y₁ is 0, 1, 2, 3, 4, 5, 6, 7, 8, 9, or 10; and L₂ is connected to L₁ at position 1 and L₂ is connected to L₃ at position 2;
L₃ is selected from a 5-12 membered heteroaromatic ring or absent;
L₄ is 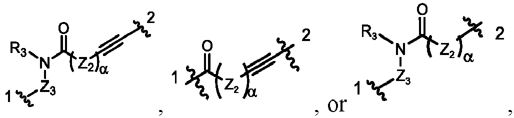 wherein Z₂ is selected from C₁₋₆ alkylene, C₂₋₁₀ alkenylene, C₂₋₁₀ alkynylene, C₃₋₈ cycloalkylene, and a 5-6 membered heteroaryl group; R₃ is selected from H and C₁₋₆ alkyl; Z₃ is absent or selected from C₁₋₆ alkylene; or, R₃ and Z₃, together with a nitrogen atom they are attached to, form a 4-8 membered heterocyclic group; α is 0, 1, 2, 3, 4, 5, or 6, and L₄ is connected to E at position 2 and L₄ is connected to L₃ at position 1;
E is 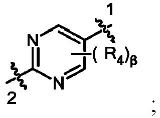 wherein, each R₄ is independently hydrogen, β is 0, 1 or 2, and E is connected to G at position 2 and E is connected to L₄ at position 1;
m₁, m₂ and m₃ each are independently 0, 1, 2, 3, 4, 5, 6, 7, 8, 9, or 10;
D is a biologically active molecule fragment; and
G is a leaving group for nucleophilic substitution reaction; preferably, G is selected from halogen, sulfonyl, sulfonate, or nitro.

2. A method for improving the product quality of an antibody-drug conjugate, wherein a 5-20 mM reducing agent is added to an antibody that is pretreated and mixed uniformly for a reduction reaction, and then a drug to be conjugated is added, and after it is stirred and rested, an acidic solution is added; a regulation solution is added to the product from the above step, column chromatography flowthrough is performed to collect a product, and the product is further subjected to ultrafiltration and/or diafiltration concentration to obtain an antibody-drug conjugate with improved product quality;
wherein the structure of the drug to be conjugated is shown in formula (I) in claim 1.

3. The method according to claim 1 or 2, wherein the drug to be conjugated is selected from the compounds shown below: 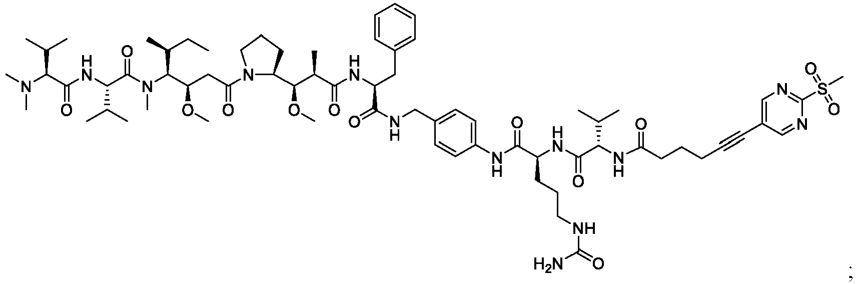 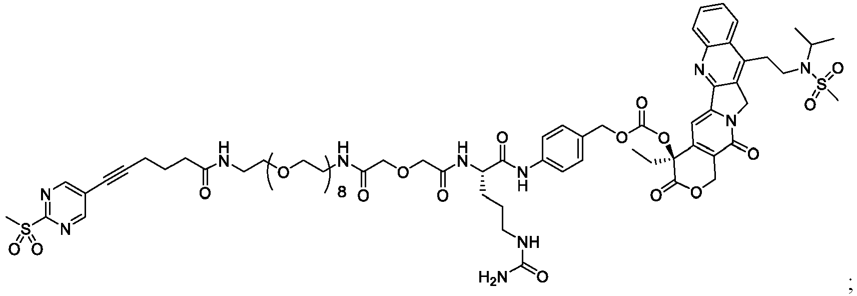 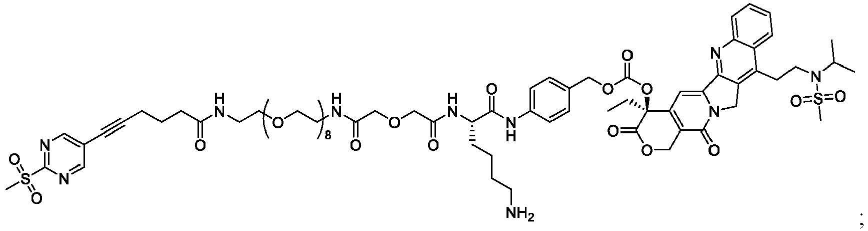 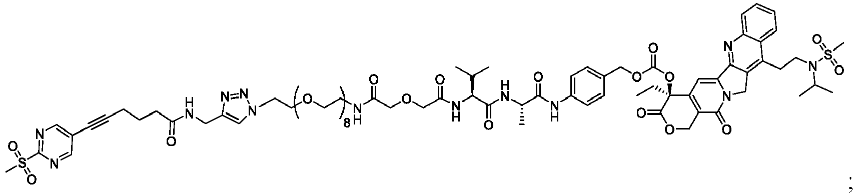 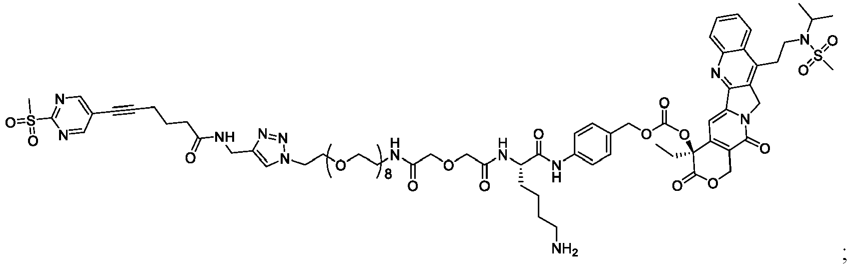 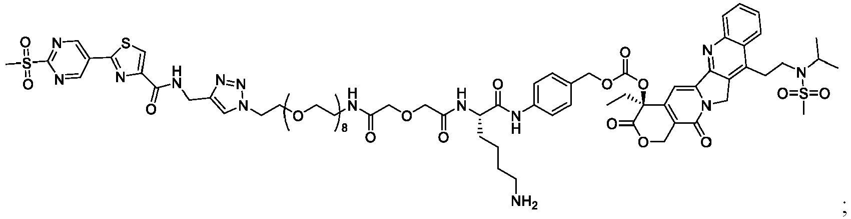 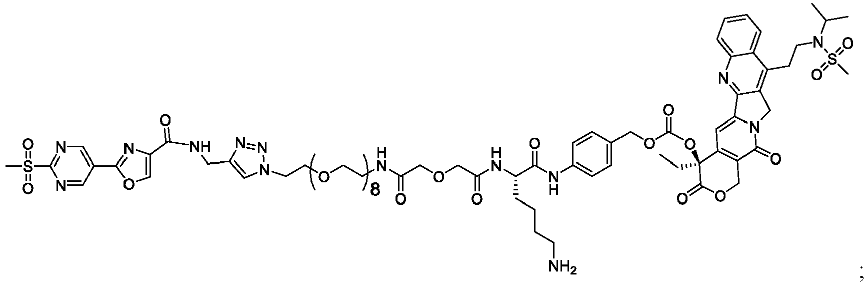 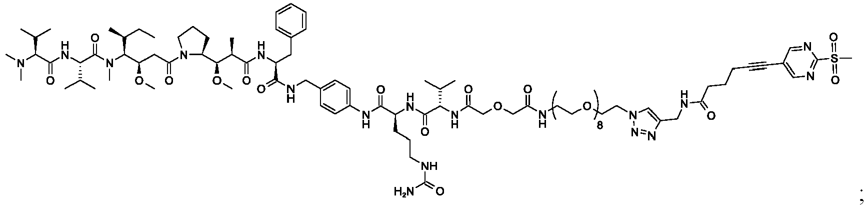 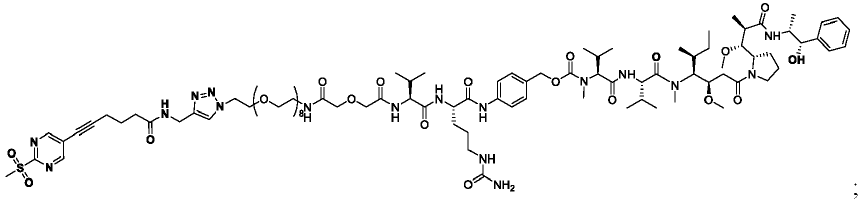 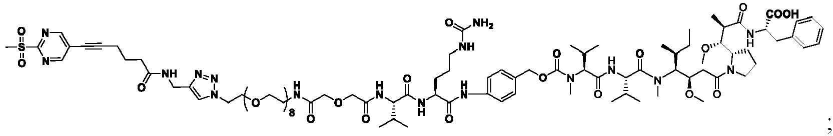 and

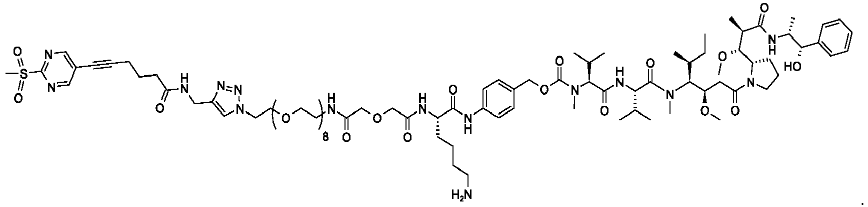

4. The method according to any one of claims 1-3, wherein the column chromatography is hydrophobic chromatography;
preferably, the hydrophobic chromatography comprises steps of equilibration and elution;
preferably, the equilibrium step is using 20 mmol/L phosphate buffer-ammonium sulfate solution as an equilibrium solution under pH 6-7 conditions, wherein the conductivity is controlled at 50-80 mS/cm.

5. The method according to claim 4, wherein the column chromatography is hydrophobic chromatography;
preferably, the hydrophobic chromatography comprises steps of equilibration and elution;
preferably, the equilibrium step is using 20 mmol/L phosphate buffer-ammonium sulfate solution as an equilibrium solution under pH 6-7 conditions, wherein the conductivity is controlled at 62-70 mS/cm.

6. The method according to any one of claims 1-5, wherein the elution step is using the same solution as the equilibrium solution as an eluent under pH 6-7 conditions, wherein the conductivity is controlled at 22-30 mS/cm.

7. The method according to any one of claims 1-6, wherein the regulation solution is an ammonium sulfate solution.

8. The method according to any one of claims 1-7, wherein the antibody-drug conjugate in step (1) is obtained by the following method: a reducing agent solution with a concentration of 5-20 mM is added to an antibody that is pretreated, and then a drug to be conjugated is added to the reaction system, and after it is stirred and rested, an acidic solution is added to obtain the antibody-drug conjugate.

9. The method according to claim 8, **characterized by** one or more of the following:
1) the reducing agent is TCEP (tris(2-carboxyethyl)phosphine);
2) the antibody is treated with the reducing agent for 10 min-2h, for example, 30 min-1h;
3) the antibody is treated with the reducing agent at room temperature; and/or, the reaction is performed at 4-37°C, for example, 18-26°C;
4) the drug to be conjugated is added to the reaction system and stirred for 5-15 min, and then rested to react, preferably, rested for 1h-12h, such as 1h-6h, 1h-4h, 1h-3h, 1.5h-3h, or 1.5h-2.5h.
5) the acidic solution is a citric acid solution or an acetic acid solution, and the acidic solution is used to adjust the pH of the reaction system to acidity; preferably, the acidic solution adjusts the pH of the reaction system to 4.5-7.0; further preferably, the acidic solution adjusts the pH of the reaction system to 6.0-7.0;
6) in the reduction reaction, the dosing concentration of the antibody is 10-25 g/L; and
7) the dosing concentration of the drug to be conjugated is 10-70 mM, preferably 50 mM.

10. The method according to claim 8 or 9, wherein the antibody pretreatment step comprises steps of adding a disodium edetate solution to the antibody and adjusting the pH using a Tris solution,
preferably, the concentration of the disodium edetate solution is 2-10 mM, and the concentration of the Tris solution is 1-3 M.

11. The method according to claim 8 or 9, wherein the antibody pretreatment step comprises steps of adding a disodium edetate solution to the antibody and adjusting the pH with a Tris solution, wherein the concentration of the disodium edetate solution is 2-10 mM, and the concentration of the Tris solution is 2 M.

12. The method according to claim 8 or 9, wherein the pretreatment step comprises steps of adding a disodium edetate solution to the antibody and adjusting the pH using a phosphate;
preferably, the phosphate is sodium phosphate; further preferably, the sodium phosphate is disodium hydrogen phosphate;
preferably, the concentration of the disodium edetate solution is 2-10 mM, and the concentration of the disodium hydrogen phosphate solution is 0.2-2 M, preferably 1 M.

13. The method according to any one of claims 1-12, wherein the antibody is further purified before pretreatment, and the purification comprises steps of chromatography selected from affinity chromatography, anion exchange chromatography, and/or cation exchange chromatography, and filtration;
preferably, the purification sequentially comprises affinity chromatography, incubation, depth filtration, anion exchange chromatography, and cation exchange chromatography, and does not comprises ultrafiltration or diafiltration.

14. The method according to any one of claims 1-13, wherein the antibody is an anti-Her2 antibody, an anti-EGFR antibody, an anti-PD-1 antibody, an anti-PD-L1 antibody, or an anti-Trop-2 antibody; preferably, the anti-Her2 The antibody is Trastuzumab or Pertuzumab, the anti-EGFR antibody is Cetuximab or Nimotuzumab, the anti-PD-1 monoclonal antibody is Nivolumab or Pembrolizumab, the anti-PD-L1 monoclonal antibody is Atezolizumab or Durvalumab, and the anti-Trop-2 antibody is Sacituzumab or Datopotamab.

15. The method according to any one of claims 1-14, wherein the drug to be conjugated is a small molecular compound with a connecting arm, the small molecular compound selected from a DNA topoisomerase inhibitor, a tubulin inhibitor, or derivatives thereof; preferably, the topoisomerase inhibitor is a topoisomerase I inhibitor or a topoisomerase II inhibitor; preferably, the topoisomerase I inhibitor is camptothecin, SN-38, irinotecan, topotecan, belotecan, rubitecan, or derivatives thereof; preferably, the topoisomerase II inhibitor is actinomycin D, doxorubicin, doxorubicin, docamicin, daunorubicin, mitoxantrone, podophyllotoxin, etoposide, or derivatives thereof; preferably, the tubulin inhibitor is vinca alkaloid, vincristine, vinblastine, paclitaxel, docetaxel, cabazitaxel, or derivatives thereof.

16. The method according to any one of claims 1-15, wherein the antibody is Sacituzumab; the structure of the drug to be conjugated is as follows: 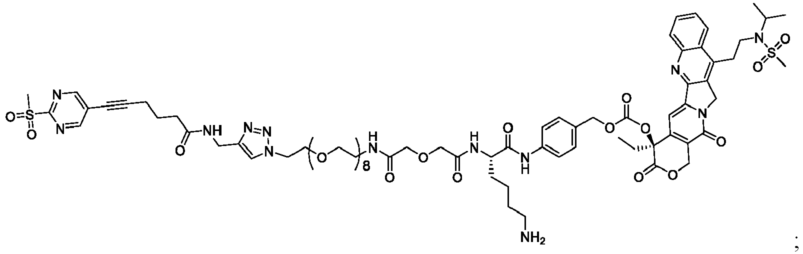 preferably, the structure of the antibody-drug conjugate is as follows: 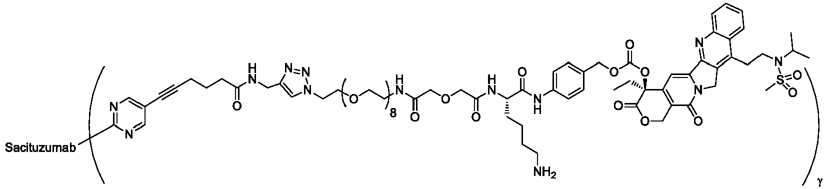 wherein, y is 1-10; preferably, y is 5-8; preferably, the connecting arm is connected to the sulfydryl group of Sacituzumab.
